# EUROPEAN PATENT SPECIFICATION

(11) **EP 0 506 420 B1**
(45) Date of publication and mention of the grant of the patent: **20.05.1998**
(21) Application number: 92302658.7
(22) Date of filing: 26.03.1992
(51) Int. Cl.: A61B 17/70, A61B 17/80

(54) **Bone fixation apparatus**
Knochenbefestigungsvorrichtung
Dispositif de fixation osseuse

(30) Priority: 27.03.1991 US 675740; 24.03.1992 US 856707
(43) Date of publication of application: 30.09.1992
(73) Proprietor: SMITH & NEPHEW, INC., Memphis, Tennessee 38116 (US)
(72) Inventor: Small, Laura, Memphis Tennessee 38111 (US); Brumfield, David, Nesbit Mississippi (US); Yuan, Hansen, Fayettevilte New York 13066 (US); Hildebrand, Bryan, Memphis Tennessee 38104 (US); Smmons, James, San Antonio Texas 78229 (US)
(74) Representative: Koepsell, Helmut, Dipl.-Ing.

(56) References cited:
- EP-A- 0 410 309
- EP-A- 0 441 084
- DE-C- 251 246
- FR-A- 1 037 262
- GB-A- 780 652
- US-A- 4 085 744
- US-A- 4 604 995
- US-A- 4 611 581
- US-A- 4 836 196

## Description

The present invention relates to surgical systems and more particularly relates to an improved bone or spinal fixation apparatus in the form of a strong and stable construct for maximum fusion augmentation with improved versatility and ease of use, and wherein an improved fine adjustment plate assembly forms a load transfer interface with an adjacent fixation bolt or screw. A countersunk interface provides some micromotion, better stress distribution and thus enhanced fatigue life.

There are a number of surgical procedures which require fixation of portions of the spine with respect to one another. Typically, bone screws are employed in the fixation of the spine. The implantation of bone screws is a surgical procedure which involves the formation of one or more surgical openings in adjacent portions of the spine, with threaded bone screws being implanted into these surgical openings. Connective structure such as rods or plates extend between the various spine members by connecting the adjacent bone screws.

An early bone fixation system was described in DE-A-251246 which shows a metal bracing plate, washers and fastening screws. The bracing plate has serrations on its upper face or within a channel formed in the plate which cooperate with corresponding serrations on the washer to hold the washer in a fixed position adjacent the plate.

An early spinal fixation system can be seen in the Lumb et al. patent (US3648691) entitled "Method of Applying Vertebral Appliance". In the Lumb patent, a method of applying a vertebral appliance for use in bridging one or more diseased or damaged vertebra uses a pair of elongated flexible multiple apertured plates having fasteners which are used to clamp the plate to opposite sides of the spinous processes being spanned. Each strap or plate is of a length adpated to span at least two spinous processes and project there beyond each end so that the fasteners can be passed both behind and in front thereof as well as through the interspinous gap there between. The apertures are located considerably closer together than adjacent processes and they are fastened to the latter in position such that at least one opening registers with each one to receive a growth or soft bony tissue that eventually extrudes therein.

The Edwards patent (US4369769) shows a spinal fixation system using elongated rods used to bridge across various portions of the spine. In the Edwards '769 patent a spinal fixation device is provided in which sleeves or spacers are placed over and around spinal rods in order to obtain a better reduction of spinal fractures or spinal deformities. These sleeves can be made in various thicknesses so that the surgeon can obtain optimum fixation in each case. The sleeves are made of any biologically compatible material.

Use of bone screws and connecting rods is also seen in the Ulrich et al. patent (US4433677) entitled "Implantable Splint for Correction Lumbosacral Spondylodesis". In the Ulrich patent a spinal distraction splint has two like anchor screws extending along respective longitudinal screw axes and adapted to be anchored in the pelvis with the axes crossing. Each of the screws has a head formed with a transverse open recess centred on respective transverse axis and with an angular array of teeth centred on and angularly spaced about the respective transverse axis.

Another patent that shows screws as part of a spinal stabiliser is the Stephens et al. patent (US4604995). In the Stephens patent a surgical implant is used for imparting stability to the thoraco-lumbar spine by fixation of the implant to the spine with segmental spinal instrumentation. The implant comprises a unitary rod having a generally rectangular configuration formed by a pair of spaced apart branches, mirror image duplicated of one another and equally spaced apart along their length.

The Steffee patent (US4611581) entitled "Apparatus for Straightening Spinal Columns" provides an apparatus to reduce the extent of displacement between adjacent vertebra in a person's spinal column and to subsequently maintain the vertebra in a reduced displacement relationship. When the apparatus is to be installed, holes are formed in the displaced vertebra and in vertebra on opposite sides of the displaced vertebra. Force transmitting members are mounted in the holes in the vertebra. A spinal plate is then positioned on the spinal column with the force transmitting members extending outwardly through the slots in the spinal plate. Nuts are tightened on the force transmitting members connected with vertebra on opposite sides of the displaced vertebra to anchor the spinal plate in place. A nut on the force transmitting member connected with the displaced vertebra is then tightened to pull the displaced vertebra to a desired position. In one embodiment, the force transmitting member has a relatively large diameter helix which engages a side wall of the hole in the displaced vertebra. In another embodiment, an insert is positioned in a hole in the displaced vertebra and expanded by the force transmitting member to securely grip the vertebra.

A device which uses clamps as opposed to bone screws is the Asher patent (US4773402) entitled "Dorsal Transacral Surgical Implant" wherein a pair of spinal engageable rods, contoured to the desired spinal column configuration are provided with a yoke and foot element being attached to the pair of rods during use.

The Sherman patent (US4887596) shows a pedicle screw for use in internal fixation of the spine comprising a shaft threaded at one end for insertion into a bone and having at the other end a yoke for receiving a rod, the yoke having a cusp adapted to bear against the rod and clamps for holding the rod against the cusp while permitting adjustment of the angle between the rod and the yoke.

A further fixation system for bones is described in EP-A-410309. That system includes a long plate or strap along which are several holes through which a screw or screws may be placed into a bone or bones. Washers are provided to interface the plate and the screw and the upper surface of the plate is notched to engage the lower edges of the washers.

One of the problems with the application of a spinal fixation system is the adjustability of the connective structures such as a plate with respect to a plurality of spaced apart bone screws.

It is thus an object of the present invention to provide a fixation system that offers a strong and stable construct for maximum fusion augmentation and yet is versatile enough for any patient and is easy to use.

One of the features of the present invention is a countersunk interface between the bone bolt and the plate. A countersunk interface is also located between a washer and nut. This improved construction provides some micromotion, better stress distribution, and thus enhanced fatigue life.

Another object of the present invention is to provide an improved spinal fixation apparatus having improved fit through the use of a fine adjustment between adjacent bolts/screws. This high resolution allows each screw and/or bolt to be placed anatomically with little manipulation to make the place fit the bolts and screws of the system.

Also, the present invention provides an improved spinal fixation system that allows the surgeon to choose the amount of rigidity needed for each patient and his particular indication.

In accordance with the present invention there is provided fixation apparatus comprising at least two fixation members adapted, at a first end, to be implanted into bone tissue at spaced apart positions, a plate member having a pair of opposed parallel peripheral edges and having an elongated slot formed therein between said edges and a load transfer member for distributing the load from each fixation member to the plate member wherein the load transfer member and the slot in the plate member are each adapted to permit placement of a portion of the fixation member therethrough and said plate member and said load transfer member are adapted to cooperate to provide means for the fine adjustment of the spacial relationship between the plate member and the load transfer member, characterised in that the load transfer member and said peripheral edges of the plate member are adapted to co-operate to provide means for the fine adjustment of the spacial relationship between the plate member and the load transfer member.

According to a preferred embodiment the apparatus of the invention comprises:
a) first fixation means comprising bone bolt means having first end portions adapted to be surgically implanted into a patient's bone tissue at first and second spaced apart positions on the bone tissue;
b) a central section of the bone bolt means having a load transfer surface;
c) the bone bolt means having a threaded section for threadably receiving a nut;
d) a plate member having upper and lower surfaces and parallel outer opposed edges with an elongated slot surrounded by a peripheral portion having said parallel outer opposed edges;
e) the edges carrying fine adjustment means extending between the upper and lower surfaces for affixing the position of the bone bolt with respect to the plate; and
f) a load transfer member interfacing the plate member and the bone bolt means, for distributing load from the bone bolt means to the plate member and including sidewall portions for engaging the fine adjustments means at the edges, and an opening in the washer for placement of a portion of the bone bolt means therethrough.

In another preferred embodiment the bone fixation apparatus, comprises:
a) bone screw means having first end portions adapted to be surgically implanted into a patient's bone tissue at a first and second spaced apart positions on the bone tissue;
b) a plate member having upper and lower surfaces and parallel outer opposed edges with an elongated slot surrounded by a peripheral portion having said parallel outer opposed edges;
c) the edges carrying fine adjustment means extending between the upper and lower surfaces for affixing the position of the bone screw with respect to the plate; and
d) load transfer member interfacing the plate member and the bone screw means for distributing load from the bone screw means to the plate member and including sidewall portions of the member means for engaging the fine adjustment means at the edges, and an opening in the member for placement of a portion of the bone screw means therethrough.

In yet a further preferred embodiment the invention is a cervical bone fixation apparatus, comprising:
a) a fixation member comprising bone screw means having first end portion adapted to be surgically implanted into a patient's cervical bone mass and at first and second spaced apart positions on the bone mass;
b) an end portion of the bone screw means having a load transfer surface;
c) a plate member having upper and lower surfaces angled with respect to each other, with an elongated plate slot surrounded by a peripheral portion having parallel outer opposed edges;
d) a load transfer washer interfacing the plate member and the bone screw means, for distributing load from the bone screw means to the plate member and e) the bone screw means being selectively supportable by the washer along a line that forms an acute angle with the upper surface of the plate member.

According to a preferred aspect of the invention the load transfer member includes a washer having a first flange that extends across the slot between the edges and a pair of intersecting flanges extending at angles thereto.

The fixation member may be provided with a screw thread at said first end to allow it to be surgically implanted into the bone tissue. Aptly the fixation member may have an end portion having a load transfer surface. The fixation member may also be provided with a threaded section for threadably receiving a nut. Suitable fixation members will be in the form of bone screws or bone bolts.

The plate member has upper and lower surfaces which may be angled or parallel with respect to each other.

The slot between the edges of the plate may be sized to accommodate a pair or more of the spaced apart, surgically implanted fixation means eg. bone screws or bone bolts.

The means for finely adjusting the special relationship between the plate member and the load transferring member may include teeth spaced along the edges. The teeth may be regularly spaced along each of the edges and will engage and co-operate with corresponding teeth on the load transfer member.

The load transfer member which aptly is the form of a washer with turned edges flanges or extensions may have an opening therein with a centre that registers with the central axis of the slot during use. The load transfer member may be adapted to support said fixation member along a line that forms an acute angle with respect to the upper surface of the plate member. Thus the axis of the opening may be at angle with respect to the plane of the member.

Suitably the load transfer member will include a recess that registers with the plate so that said member slides upon the plate at the recess.

preferably the load transfer member has three intersecting flange portions including a centre flange and two side flanges that are spaced apart and positioned to bear against the edges of the plate member.

The load transfer washer opening may be spherically shaped to receive a nut having a spherical lower surface.

In a preferred arrangement the fixation member comprises a bone bolt having a central section bearing a longitudinally extending, shaped load transfer surface and having a second threaded section for threadably receiving a nut together uith a bone plate member having upper and lower surfaces and parallel outer opposed edges with an elongated slot surrounded by a peripheral portion having said parallel outer opposed edges the plate lower surface being countersunk at the slot to fit the bone bolt load transfer surface, and the slot being sized to receive the second threaded section of the bone bolt.

The bone plate member may carry closely spaced fine adjustment means extending between the upper and lower surfaces for affixing the position of the bone bolt with respect to the plate and including several adjustment positions per the thickness of the bone bolt.

The nut associated with the bolt may have a curved annular lower surface that registers with an opening in the load transfer member which opening has a centre that registers with the central axis of the slot during use.

The nut may be provided with a spherical lower surface and the load transfer member opening is spherically shaped to receive the nut.

The opening in the load transfer member may have a surrounding hemispherical portion that receives a corresponding hemispherical portion of the nut.

The slot may have a lower beveled surface and the bone bolt a corresponding surface that abuts the beveled surface for load transfer.

In those embodiments wherein the fixation member is a bone screw, the load transfer member includes a hemispherical surface that registers with the load transfer surface of the bone screw.

In a preferred form of a cervical bone fixation system in accordance with the invention the system will comprise:
a) one or more bone screws for example a pair of bone screws having first end portions adapted to be surgically implanted into a patient's cervical bone mass at first and second spaced apart positions on the cervical bone mass;
b) an upper portion of each bone screw having a shaped load transfer surface;
c) the bone screws each having a threaded section for engaging the cervical bone mass of the patient; and
d) a bone plate member having an asymmetrical cross-section, eg. having upper and lower planar surfaces that form an acute angle, and parallel outer opposed edges, with an elongated slot surrounded by a peripheral portion having said parallel outer opposed edges;
e) the plate slot being an angled slot that is offset by an acute angle with respect to the plate upper surface; and
f) a load transfer member that is selectively and removably connectable to the plate member, the washer having a load transfer surface that engages the load transfer surface of the bone screw during use.

The load transfer member may be in the form of a washer having a first flange that extends across the slot between the edges, and a pair of intersecting flanges extending at angles thereto.

Each bone screw may have a first threaded end portion adapted to be surgically implanted into a patient's cervical bone mass and may have a load transfer surface at its upper end portion member and the bone screw for distributing load from and the load transfer member eg. washer has a countersunk opening for receiving the bone screw upper end portion load transfer surface, and an opening for placement of a portion of the bone screw therethrough; and wherein the opening is adapted to allow the bone screw to penetrate the washer at an acute angle with respect to the plate upper surface.

For systems designed for cervical bone fixation the plate is provided with a slot that is perpendicular to the posterior face through which a screw is placed in the lateral mass using the Roy-Camille technique. The plate also has a slot that is offset by an acute angle such as fifteen degrees (20° off vertical with respect to the cross section of the plate). A screw can then be placed through the angled slot into the lateral mass using the Magerl technique.

The angled slot can also be extended at each end of the spinal plate (cephalad/caudad) allowing the screw to be placed thirty degrees cephalad when the screw is placed using the Magerl technique.

Both ends of the plate can have slot extensions (as mentioned in the previous paragraph), thus making the plate reversible, that is, one plate works on both left and right sides of the spine.

A washer (load transfer member) co-operates with and attaches to the plate at a toothed interface. The washer is designed to fit over the plate and mate with the teeth in the plate. The washer is placed after the plate is positioned on the lateral mass. The washer has an asymmetrical cross section that corresponds to the plate. The washer can have three different hole configurations for example thus requiring three separate parts (namely right, left, and neutral).

The right and left sides can have a hole through the central beam member of the washer. The right hole can be at twenty degrees lateral (right) and thirty degrees cephalad.

The left hole can be at twenty degrees lateral (left) and thirty degrees cephalad. Both the right and left washer can be used with th Magerl technique for placing the screw.

The neutral washer can have a hole placed perpendicular to the central body, allowing the screw to be placed straight into the lateral mass using the Roy-Camille technique. All three washers can have a hole with a spherical countersunk portion. As an example, the washers can have a 3.5 millimetre hole for accommodating the 3.5 millimetre major thread diameter of the bone screw which has a spherically-shaped head.

For a further understanding of the nature and objects of the present invention, reference should be made to the following detailed description taken in conjunction with the accompanying drawings, in which like parts are given like numerals, and wherein:
Figures 1 and 1D and 4 are perspective exploded views of the preferred embodiment of the apparatus of the present invention;
Figures 1A, 1B and 1C are sectional fragmentary views of the preferred embodiment of the apparatus of Figure 1;
Figures 2 and 2A are partial perspective view elements of the apparatus of the present invention;
Figures 3, 3A and 3B are schematic views illustrating the preferred embodiments of the apparatus of the present invention in use as part of spinal or cervical fixation systems;
Figure 4A is a sectional, fragmentary view of the of the apparatus shown in Figure 4;
Figure 5 is a fragmentary view of the apparatus shown in figure 1D;
Figure 6 is a front view of an element shown in Figure 1D illustrating a right side orientation of the washer portion;
Figure 7 is a side view of the rasher shown in Figure 6;
Figure 8 is a front view of the washer portion showing a left orientation;
Figure 9 is a side view of the rasher shown in Figure 8;
Figure 10 is a frontal view of the washer portion showing a neutral orientation; and
Figure 11 is a side view of the washer in Figure 10.

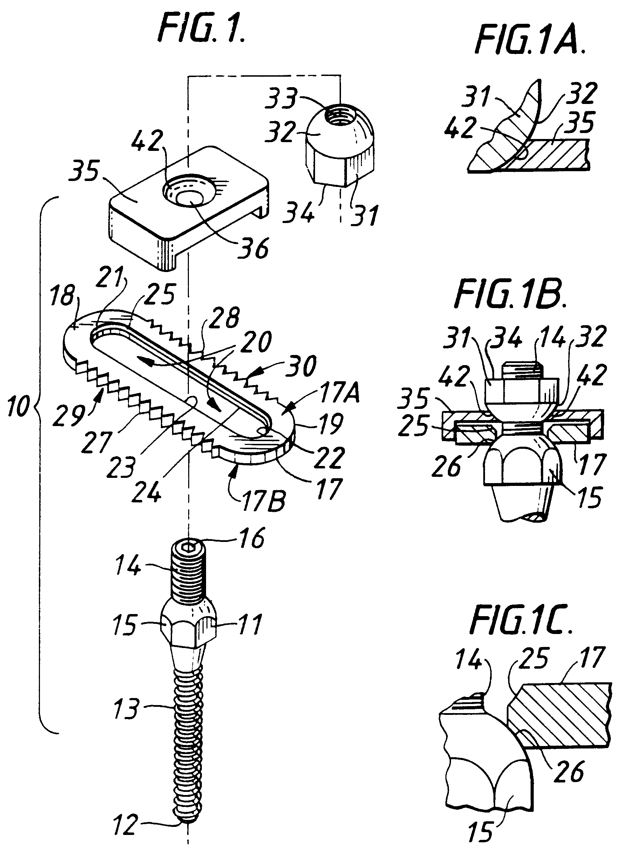

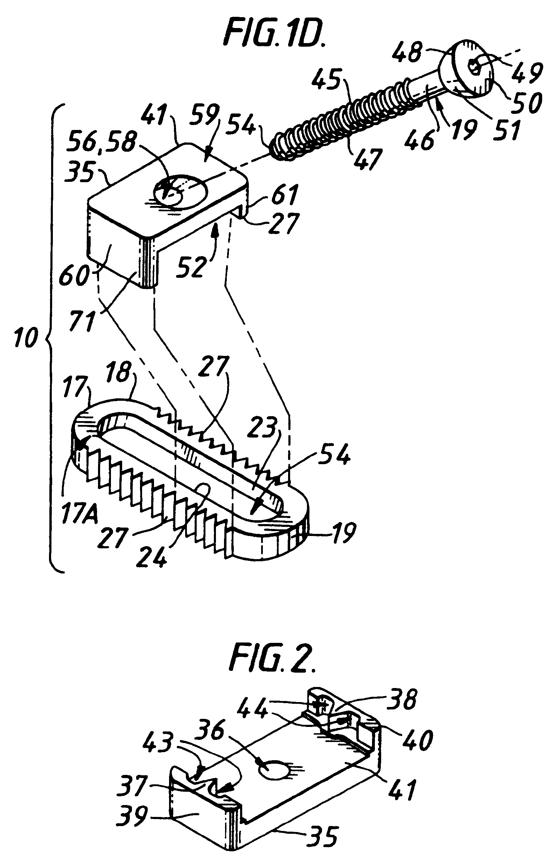

Figures 1 and 1A-1C illustrate apparatus of the present invention designated generally by the numeral 10. In Figure 1 there can be seen bone bolt 11 having a shank portion 12 with a coarse thread 13 thereon. At the upper end portion of bone bolt 11, a fine thread 14 is provided. The middle portion 15 of bone bolt 11 is in the form of a hexagonal bolt which can be formed as an integral portion of the bone bolt 11. A hexagonal tool socket 16 is preferably provided at the upper end portion of bone bolt 11, for receiving an allen wrench or the like for installation of the screw into a surgical opening.

Plate 17 includes an upper surface 17A and a lower surface 17B. The plate 17 is elongated and provides curved end portions 18, 19. A longitudinally extending slot 20 includes semicircular end portions 21, 22. Slot 20 is surrounded by parallel side walls 23, 24. The slot 20 includes an upper beveled edge 25 and a lower beveled edge 26 which communicate with the slot at the top 17A and bottom 17B of plate 17. The beveled edges 25, 26 can interface with similarly shaped surfaces of the bone bolt 11 mid portion 15 (Figure 1B).

The outer peripheral surface of plate 17 includes a plurality of spaced apart teeth 27, 28 along the sides of plate 17 (Figure 1) which define an adjustment distance of for example just a few millimetres. Thus, spaces 29, 30 are provided between teeth 27, 28 for receiving projections 37, 38 of load washer 35. The washer 35 includes side walls 39, 40 which are spaced apart and which connect to the plate transverse flange member 41.

An opening 36 in washer 35 includes a hemispherical concave surface 42 that is similarly shaped to the hemispherical convex surface 32 of nut 31. Nut 31 includes internal threads 33 that engage the fine threads 14 of the bone bolt 11 and an upper flat surface 34.

Projections 37, 38 are correspondingly shaped to register the spaces or recesses 29, 30 between teeth 27, 28. Each projection 37, 38 has a pair of recesses 43, 44 on the sides thereof.

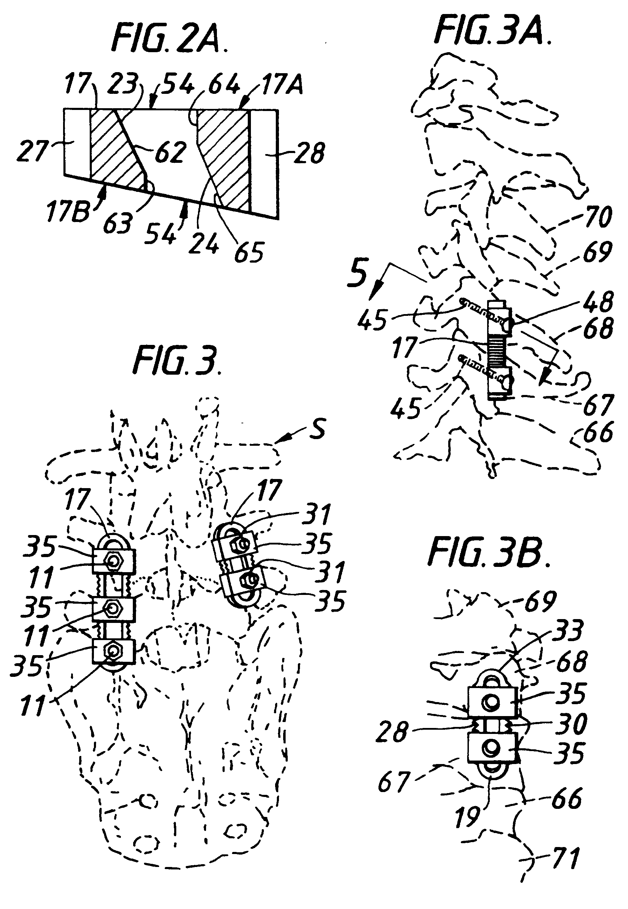

Figure 3 illustrates a pair of spaced apart plates 17 illustrating the attachment of bone bolts 11 and load transfer washers 35 as part of an overall spinal fixation system. Also illustrated is the nut 31 which threadably engages the top of the bone bolt 11. In phantom lines, the spine of the patient is illustrated generally by the letter S. It should be understood however that the surgeon can select surgical openings at particular locations to which bone bolts 11 are to be affixed and can further custom select the particular plate 17 including a particular length to accommodate one or more bone screws as part of the spinal fixation system.

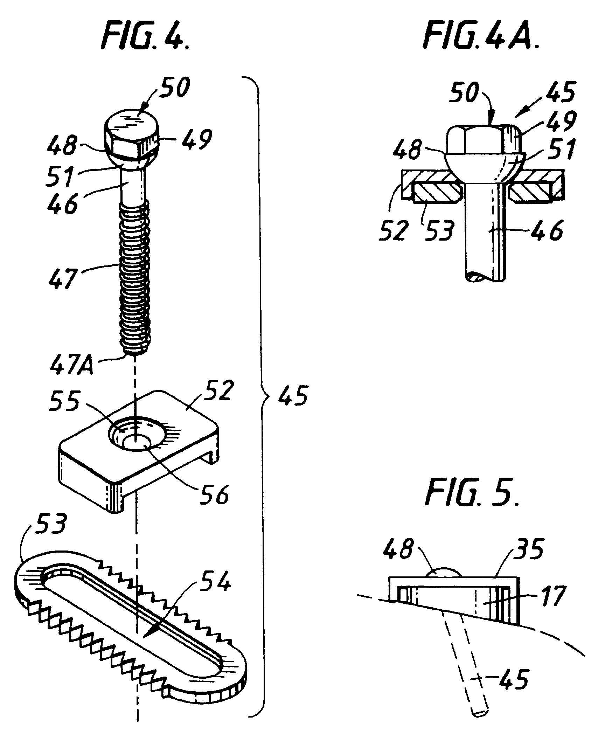

In Figures 4 and 4B, a second alternate embodiment embodiment of the apparatus of the present invention is shown designated generally by the numeral 45. In Figure 4, there can be seen bone screw 45 having a shank 46 portion with a coarse thread 47 thereon. At the upper end portion of bone screw 45, an enlarged head portion 48 is provided that includes a hexagonal portion 49 having a flat upper surface 50, and communicating with a hemispherically shaped lower surface 51 that can be integrally formed with hexagonal portion 49 and with shank 46 (Figure 4A). The flat upper surface 50 could have an internally threaded portion for connecting with a stabilising bar. Coarse threads 47 extend to the lower end portion of shank 46, terminating at 47A, as shown in Figure 4.

Washer 52 can be of the same construction as the washer 35 described with regard to the preferred embodiment of Figures 1-3. Similarly, the plate 53 could be the same construction as the plate 57 described above and with regard to the preferred embodiment of Figures 1-3.

In Figure 4A, a partial elevational view of bone screw 45 is shown, illustrating the upper end portion of the screw 45 including the shank 46 and head 48 portions in operating position with plate 54 and washer 52. The plate 53 provides a slot 54, and the washer 52 provides a hemispherical socket 55 communicating with and extending around opening 56. In this manner, the hemispherical surface 51 of bone screw 45 nests in and registers with the hemispherical concave surface 55 of washer 52. Bone screw 45 shank 46 passes through opening 56 and slot 54.

Figure 1D shows an alternative embodiment of the present invention designated generally by the numeral 10. Bone fixation apparatus 10 includes a bone screw 45 having an elongated shank 46 with an enlarged head portion 48 affixed to one end of the shank 46. The enlarged head 48 has a hemispherical surface 51 on the undersideof the head 48 and a top surface 50. A tool receptive socket such as hexagonal socket 49 is provided on the flat top surface 50, so that the bone screw 45 can be rotated using an alien wrench or other such tool or instrument.

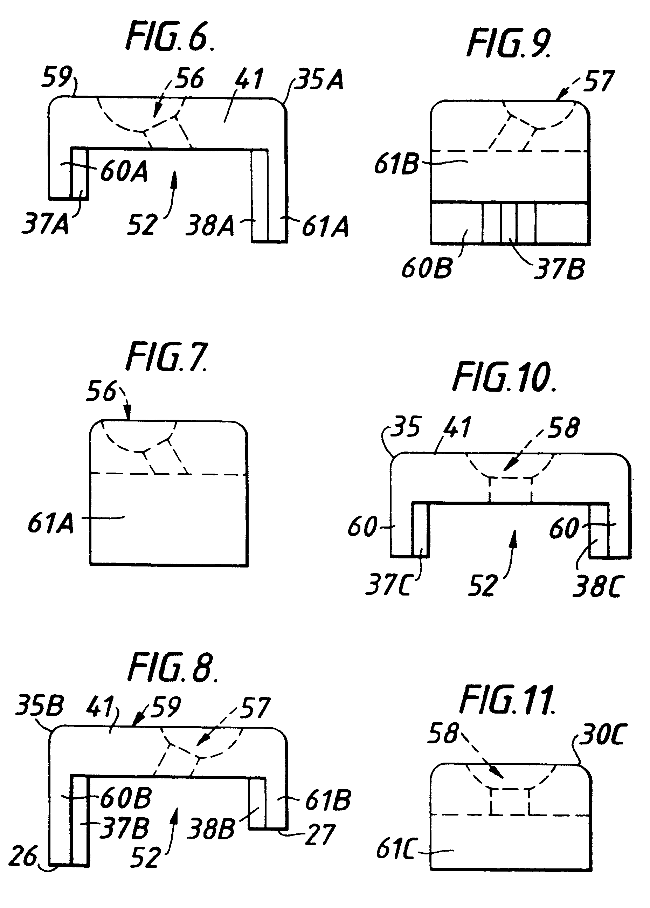

The lower tip 54 of the bone screw 45 communicates with a helical thread 47 that begins at lower tip 50 and terminates at cylindrical surface 55. The cylindrical surface 55 and hemispherical surface 51 interface with washer 35 at opening 56-58 (see Figures 6-11) as will be described more fully hereinafter.

Washer 35 provides a transverse plate member 41 having an upper surface 59. Extending downwardly from transverse plate member 41 is a pair of spaced apart plate members in the formof sidewalls or flanges 60, 61.

Figures 6 and 7 show the washer 35A having sidewalls/flange members 60A and 61A. The embodiment of Figures 8 and 9 provide sidewall or flange members 60B and 61B. The embodiment of Figures 10 and 11 illustrate sidewall or flange members 60C and 61C. The washer 35 is designed to fit over plate member 17 and mate with the teeth 27, 28 of the plate 17. The washer 35 is placed after the plate 17 is positioned on the left or right side of the lateral mass of cervical bone tissue.

The washer 35 can have an asymmetrical cross section that corresponds to the asymmetrical cross section of the plate 17. Washers 35A, 35B and 35C are provided that will have three hole configurations, as shown in drawing Figures 6 and 7 (right side version), Figures 8 and 9 (left side version) and Figures 10 and 11 (neutral version). Thus, the three hole configurations require three separate parts, namely right (35A), left (35B), and neutral (35C).

The right (35A) and left (35B) sizes will have a hole or opening 56, 57 respectively through the transverse plate member 41 as show in figures 6-7, 8-9. In the case of the embodiments of Figures 8-9, the opening 56 will be at a twenty degree (20°) lateral (right) and thirty degree (30°) cephalad. The left hole embodiment of Figures 8-9 place the hole or opening 57 at twenty degrees (20°) lateral (left) and thirty degrees (30°) cephalad. Both the right and left washer embodiments 35A, 35B of Figures 6-7 and 8-9 respectively will be used with the Magerl technique for placing the bone screw 11. The neutral washer of Figures 10-11, designated by the numeral 35C will have an opening or hole 58 placed perpendicualr to the central body, allowing the screw 45 to be placed straight into the lateral bone mass using the Roy-Camille technique. All three washers 35A, 35B, 35C can have a 3.5 millimetre hole witha spherical countersink, for example.

A recess 52 between the sidewall/flange members 60-61 is sized to receive the cross sectional area of plate member 17. As shown in Figure 2A plate member 17 has an upper surface 17A with a slot 54 therethrough. The plate 17 provides end portions 18, 19 and slot sidewall 23. The slot sidewall 23 includes a pair of angled surfaces 62, 63 while the opposite slot sidewall 24 includes angled surfaces 64, 65.

The lower surface 17B of the plate member 17 forms an acute angle with respect to the upper surface 17A of the plate member 17. In Figures 3A,3B and 5, the apparatus 10 of the present invention is shown as installed in the cervical bone tissue, the cervical bone tissue being designated by the numerals 66-71.

## Claims

1. Bone fixation apparatus (10) comprising at least two fixation members (11) adapted, at a first end (12) to be implanted into bone tissue at spaced apart positions, a plate member (17) having a pair of opposed parallel peripheral edges and having an elongated slot (20) formed therein between said edges and a load transfer member (35) for distributing the load from each fixation member (11) to the plate member (17) wherein the load transfer member (35) and the slot (20) in the plate member (17) are each adapted to permit placement of a portion of the fixation member (11) therethrough and said plate member and said load transfer member are adapted to cooperate to provide means for the fine adjustment of the spacial relationship between the plate member and the load transfer member characterised in that the load transfer member (35) and said peripheral edges of the plate member (17) are adapted to co-operate to provide means for the fine adjustment of the spacial relationship between the plate member and the load transfer member

2. Apparatus according to claim 1 wherein the fixation member (11) is provided with a screw thread (13) at said first end.

3. Apparatus according to either claim 1 or claim 2 wherein said fixation member (11) has an end portion (48) having a load transfer surface (51).

4. Apparatus according to claim 1 or claim 2 wherein said fixation member has a threaded section (14) for threadably receiving a nut (31).

5. Apparatus according to any one of claims 1 to 4 wherein said plate member (17) has upper and lower surfaces (17A, 17B) angled with respect to each other.

6. Apparatus according to claim 5 wherein said load transfer member (35) is adapted to support said fixation member along a line that forms an acute angle with respect to the upper surface (17A) of the plate member.

7. An apparatus as claimed in claim 1, wherein at least one said fixation member (11) comprises a bone bolt having a central section (15) which includes a load transfer surface, and a threaded section (14) for threadably receiving a nut (31).

8. A bone fixation apparatus as claimed in any of claims 1-7, wherein said parallel, opposed, peripheral edges carry fine adjustment means extending between the upper and lower surfaces for affixing the position of the fixation member with respect to the plate.

9. An apparatus as claimed in any of claims 1-8, wherein said load transfer member (35) includes sidewall portions (39,40) for engaging the fine adjustment means at said peripheral edges and an opening (36) for placement of a portion of said fixation member (11) therethrough.

10. Apparatus according to any one of claims 1 to 9 wherein the load transfer member includes a washer (35) having a first flange that extends across the slot between the edges and a pair of intersecting flanges extending at angles thereto.

11. Apparatus according to any one of claims 1 to 10 wherein the slot (20) is sized to accommodate a pair of spaced apart and surgically implanted fixation means (11;45).

12. Apparatus according to any one of claims 1 to 10 wherein the fine adjustment means includes teeth (27;28) spaced along the edges.

13. Apparatus according to claim 12 wherein the fine adjustment means includes regularly spaced teeth (27;28) spaced along each of the edges and corresponding teeth (37;38) on the load transfer member (35;52) for engaging the teeth (27;28) of the edges.

14. Apparatus according to any one of claims 1 to 13 wherein the load transfer member (35;52) has an opening (36;56;57;58) therein with a centre that registers with the central axis of the slot (20;54) during use.

15. Apparatus according to any one of claims 1 to 14 wherein the load transfer member (35;52) includes a recess that registers with the plate so that said member (35;52) slides upon the plate (17) at the recess.

16. Apparatus according to any one of claims 1 to 15 wherein the load transfer member has three intersecting flange portions including a centre flange (41) and two side flanges (60;61) that are spaced apart and positioned to bear against the edges of the plate member (17).

17. Apparatus according to any one of claims 1 to 15 further comprising a nut (31) with a spherical lower surface (32) and the load transfer washer opening (42) is spherically shaped to receive the nut.

18. Apparatus according to any one of claims 1 to 17 wherein the fixation member (11) comprises a bone bolt having a central section (15) bearing a longitudinally extending, shaped load transfer surface and a second threaded section for threadably receiving a nut (31); and wherein said plate member has upper and lower surfaces and parallel outer opposed edges with an elongated slot surrounded by a peripheral portion having said parallel outer opposed edges and the plate lower surface is countersunk (26) at the slot (20) to fit the bone bolt load transfer surface, the slot (20) being sized to receive the second threaded section of the bone bolt.

19. Apparatus according to claim 18 wherein the edges of the bone plate member (17) carry closely spaced fine adjustment means (27;28) extending between the upper and lower surfaces for affixing the position of the bone bolt with respect to the plate and the spacing of said means allows several adjustment positions across the thickness of the bone bolt.

20. Apparatus according to claim 18 or 19 further comprising a nut (31) with a curved annular lower surface (32) and wherein the load transfer member (35) has an opening (36) therein with a centre that registers with the central axis of the slot during use, and the opening is shaped corresponding to the nut (31) for receiving the nut.

21. Apparatus according to claim 17 or 18 further comprising a nut (31) with a spherical lower surface (32) and the load transfer member opening (56;57;58) is spherically shaped to receive the nut.

22. Apparatus according to any one of claims 19 to 21 wherein the opening (56;57;58) has a surrounding hemispherical portion (55) that receives a corresponding hemispherical portion (32) of the nut.

23. Apparatus according to any one of the claims 18 to 22 wherein the slot (20) has a lower beveled surface (26) and the bone bolt has a corresponding surface that abuts the beveled surface for load transfer.

24. Apparatus according to any one of claims 1 to 6 or 8 to 17 wherein the fixation member is a bone screw.

25. Apparatus according to claim 24 wherein the load transfer member (35;52) includes a hemispherical surface (55) that registers with the load transfer surface (51) of the bone screw means.

26. Apparatus according to either of claims 24 or 25 wherein the load transfer member (52) has an angled opening (56;57) that receives the bone screw (45) in an angled position with respect to the plate upper surface.

27. An apparatus as claimed in any of claims 24-26 wherein the bone plate member has upper (17A) and lower (17B) planar surfaces that form an acute angle, and parallel outer opposed edges, with an elongated slot (20) surrounded by a peripheral portion having said parallel outer opposed edges and the plate slot (20) is an angled slot that is offset by an acute angle with respect to the plate upper surface (17A).

28. A bone fixation apparatus according to claim 27, comprising:
a) a pair of bone screws (45) each having a first end portion (12) adapted to be surgically implanted into a patient's cervical bone mass at first and second spaced apart positions on the bone mass;
b) a plate member (17) having upper and lower surfaces and parallel outer opposed edges with an elongated longitudinal slot (20) surrounded by a peripheral portion having said parallel outer opposed edges and the plate being asymmetrical in transverse cross section;
c) the edges carrying fine adjustment means (27) extending between the upper (17A) and lower (17B) surfaces for affixing the position of the bone screw (45) with respect to the plate(17); and
d) a load transfer washer (35) interfacing the plate member (17) and the bone screws (45) for distributing load from the bone screws to the plate member and including sidewall portions (39,40) of the washer for engaging the fine adjustment means at the edges, and an opening in the washer (35) for placement of a portion of each bone screw (45) therethrough at an angle with respect to the plate upper surface (17A).

29. A bone fixation apparatus as claimed in any of claims 24-26 wherein the plate member (17) has an asymmetrical cross section, upper and lower surfaces, and parallel outer opposed edges with an elongated slot surrounded by a peripheral portion having said parallel outer opposed edges; the load transfer washer having a countersunk opening for receiving the bone screw upper end portion load transfer surface, and for placement of a portion of the bone screw therethrough; said load transfer washer opening (36) being adapted to allow the bone screw to penetrate the washer at an acute angle with respect to the plate upper surface (17A).

## Patentansprüche

1. Knochen-Fixierungsvorrichtung (10) mit wenigstens zwei Fixierungselementen (11), die so ausgebildet sind, daß sie an einem ersten Ende im Knochengewebe in Positionen implantierbar sind, die einen Abstand voneinander aufweisen, einem Plattenelement (17) mit einem Paar einander gegenüberliegender und paralleler Umfangskanten und einem darin ausgebildeten langgestreckten Schlitz (20) zwischen diesen Kanten, und einem Lastübertragungselement (35), um die Belastung von jedem Fixierungselement (11) zum Plattenelement (17) zu verteilen, wobei das Lastübertragungselement (35) und der Schlitz (20) im Plattenelement jeweils so ausgebildet sind, daß sie das Positionieren eines Bereiches des Fixierungselementes (11) durch dieses hindurch ermöglichen, und das Plattenelement und das Lastübertragungselement zusammenwirken können, um eine Feineinstellung der räumlichen Beziehung zwischen dem Plattenelement und dem Lastübertagungselement zu ermöglichen, **dadurch gekennzeichnet**, daß das Lastübertragungselement (35) und die Umfangskanten des Plattenelementes (17) zusammenwirken können, um Mittel für die Feineinstellung der räumlichen Beziehung zwischen dem Plattenelement und dem Lastübertragungselement zu bilden.

2. Vorrichtung nach Anspruch 1, bei welcher das Fixierungselement (11) an dem ersten Ende mit einem Schraubgewinde (13) versehen ist.

3. Vorrichtung nach Anspruch 1 oder 2, bei welcher das Fixierungselement (11) einen Endabschnitt (48) mit einer Lastübertragungsfläche (51) aufweist.

4. Vorrichtung nach Anspruch 1 oder 2, bei welcher das Fixierungselement einen Gewindeabschnitt (14) zur Aufnahme einer Gewindemutter (31) aufweist.

5. Vorrichtung nach einem der Ansprüche 1 - 4, bei welcher das Plattenelement (17) obere und untere Oberfläche (17A, 17B) aufweist, die unter einem Winkel zueinander verlaufen.

6. Vorrichtung nach Anspruch 5, bei welcher das Lastübertragungselement (35) so ausgebildet ist, daß es das Fixierungselement entlang einer Linie stützen kann, die einen spitzen Winkel in bezug auf die obere Fläche (17A) des Plattenelementes bildet.

7. Vorrichtung nach Anspruch 1, bei welcher wenigstens ein Fixierungselement (11) einen Knochenbolzen mit einem mittleren Abschnitt (15), der mit einer Lastübertragungsfläche versehen ist, und einen mit Gewinde versehenen Abschnitt (14), auf dem eine Gewindemutter (31) aufschraubbar ist, aufweist.

8. Knochen-Fixierungsvorrichtung nach einem der Ansprüche 1 - 7, bei welchem die parallelen, einander gegenüberliegenden Umfangskanten Feineinstellungsmittel tragen, die sich zwischen oberer und unterer Fläche erstrecken, um die Position des Fixierungselementes in bezug auf die Platte festzulegen.

9. Vorrichtung nach einem der Ansprüche 1 - 8, bei welcher das Lastübertragungselement (35) Seitenwand-Bereiche, die mit dem Feineinstellmittel an den Umfangskanten in Eingriff bringbar sind, und eine Öffnung (36) aufweist, um einen Bereich des Fixierungselementes (11) durch diese hindurchgehend anzuordnen.

10. Vorrichtung nach einem der Ansprüche 1 - 9, bei welcher das Lastübertragungsmittel eine Scheibe (35), die mit einem ersten Flansch versehen ist, der sich quer über den Schlitz zwischen den Kanten erstreckt, und ein Paar schneidender Flanschen aufweist, die sich jeweils unter Winkel dazu erstrecken.

11. Vorrichtung nach einem der Ansprüche 1 - 10, bei welcher der Schlitz (20) so bemessen ist, daß er ein Paar einen Abstand voneinander aufweisender und chirurgisch implantierter Fixierungsmittel (11; 45) aufnimmt.

12. Vorrichtung nach einem der Ansprüche 1 - 10, bei welcher das Feineinstellmittel Zähne (27, 28) aufweist, die entlang den Kanten in Abständen voneinander angeordnet sind.

13. Vorrichtung nach Anspruch 12, bei welcher das Feineinstellmittel in regelmäßigen Abständen angeordnete Zähne (27; 28), die entlang jeder der Kanten in Abständen angeordnet sind, und entsprechende Zähne (37; 38) am Lastübertragungsmittel (35; 52) umfaßt, die mit den Zähnen (27; 28) der Kanten in Eingriff bringbar sind.

14. Vorrichtung nach einem der Ansprüche 1 - 13, bei welcher das Lastübertragungselement (35; 52) eine Öffnung (36; 56; 57; 58) darin hat mit einem Mittelpunkt, der mit der Mittelachse des Schlitzes (20; 54) bei Benutzung übereinstimmt.

15. Vorrichtung nach einem der Ansprüche 1 - 14, bei welcher das Lastübertragungselement (35; 52) eine Ausnehmung aufweist, die mit der Platte übereinstimmt, so daß das Element (35; 52) auf der Platte (17) an der Ausnehmung gleitet.

16. Vorrichtung nach einem der Ansprüche 1 - 15, bei welcher das Lastübertragungselement drei sich schneidende Flanschbe-reiche einschließlich eines Mittelflansches (42) und zweier Seitenflansche (60; 61) aufweist, die einen Abstand voneinander haben und so angeordnet sind, daß sie an den Kanten des Plattenelementes (17) anliegen.

17. Vorrichtung nach einem der Ansprüche 1 - 15 mit einer Mutter (31) mit einer kugelförmigen unteren Fläche (32), wobei die Öffnung (42) in der Lastübertragungsscheibe kugelförmig ausgebildet ist, um die Mutter aufzunehmen.

18. Vorrichtung nach einem der Ansprüche 1 - 17, bei welcher das Fixierungselement (11) eine einen zentralen Abschnitt (15) aufweisenden Knochenschraube hat und der zentrale Abschnitt eine sich longitudinal erstreckende geformte Lastübertragungsfläche und einen zweiten Abschnitt zum Aufnehmen einer Gewindemutter (31) trägt und das Plattenelement untere und obere Fläche und parallele äußere, einander gegenüberliegende Kanten und einen langgestreckten Schlitz aufweist, welcher von einem Umfangsbereich umgeben ist, der die parallelen äußeren, einander gegenüberliegenden Kanten aufweist, und die untere Fläche der Platte am Schlitz (20) versenkt (26) ist, daß er an die Lastübertragungsfläche der Knochenschraube angepaßt ist, wobei der Schlitz (20) so bemessen ist, daß er den zweiten Gewindeabschnitt der Knochenschraube aufnimmt.

19. Vorrichtung nach Anspruch 18, bei welcher die Kanten des Knochenplattenelementes (17) in kleinen Abständen voneinander angeordnete Feineinstellmittel (27; 28) tragen, die sich zwischen oberer Fläche und unterer Fläche erstrecken, um die Position der Knochenschraube in bezug auf die Platte zu fixieren, und die Abstände zwischen den Mitteln einige Einstellpositionen über die Dicke der Knochenschraube ermöglichen.

20. Vorrichtung nach Anspruch 18 oder 19, die weiterhin eine Mutter (31) mit einer gebogenen ringförmigen unteren Fläche (32) aufweist, wobei das Lastübertragungselement (35) eine Öffnung (36) hat mit einer Mitte, die bei Benutzung zur Mittelachse des Schlitzes ausgerichtet ist, und die Öffnung entsprechend der Mutter (31) zur Aufnahme derselben geformt ist.

21. Vorrichtung nach Anspruch 17 oder 18, die weiterhin eine Mutter (31) mit einer kugelförmigen unteren Fläche (32) aufweist, wobei die Öffnung (56; 57; 58) des Lastübertragungselementes zur Aufnahme der Mutter kugelförmig ausgebildet ist.

22. Vorrichtung nach einem der Ansprüche 19 - 21, bei welcher die Öffnung (56; 57; 58) einen umgebenden halbkugelförmigen Bereich (55) aufweist, der einen entsprechenden halbkugelförmigen Bereich (32) der Mutter aufnimmt.

23. Vorrichtung nach einem der Ansprüche 18 - 22, bei welcher der Schlitz (20) eine untere abgeschrägte Fläche (26) und die Knochenschraube eine entsprechende Fläche aufweist, die an der abgeschrägten Fläche für die Lastübertragung anliegt.

24. Vorrichtung nach einem der Ansprüche 1 - 6 oder 8 - 17, bei welcher das Fixierungselement eine Knochenschraube ist.

25. Vorrichtung nach Anspruch 24, bei welcher das Lastübertragungselement (35; 52) eine halbkugelförmige Fläche (55) aufweist, die mit der Lastübertragungsfläche (51) des Knochenschrauben-Mittels übereinstimmt.

26. Vorrichtung nach einem der Ansprüche 24 oder 25, bei welcher das Lastübertragungselement (52) eine abgewinkelte Öffnung (56; 57) aufweist, die die Knochenschraube (54) in einer abgewinkelten Position in bezug auf die obere Fläche der Platte aufnimmt.

27. Vorrichtung nach einem der Ansprüche 24 - 26, bei welcher das Knochenplattenelement eine obere (17A) und eine untere (17B) ebene Oberfläche hat und beide Oberflächen einen spitzen Winkel einschließen und parallele äußere einander gegenüberliegende Kanten mit einem langgestreckten Schlitz (40), der von einem Umfangsbereich umgeben ist, der die parallelen äußeren, einander gegenüberliegenden Kanten aufweist, am Knochenplattenelement vorgesehen sind, wobei der Plattenschlitz (20) ein abgewinkelter Schlitz ist, der durch einen spitzen Winkel gegenüber der oberen Fläche (17A) der Platte abgesetzt ist.

28. Knochen-Fixierungsvorrichtung nach Anspruch 27 mit
a) einem Paar Knochenschrauben (45), von denen jede einen ersten Abschnitt (12) aufweist, der an zwei einen Abstand voneinander aufweisenden Positionen an der zervikalten Knochenmasse eines Patienten in diese chirurgisch implantierbar ist,
b) einem Plattenelement (17) mit oberer und untere Fläche und parallelen äußeren, einander gegenüberliegenden Kanten mit einem langgestreckten Längsschlitz (20), der von einem Umfangsbereich umgeben ist, welcher die parallelen äußeren, einander gegenüberliegenden Kanten aufweist, wobei die Platte im Querschnitt asymmetrisch ist, und
c) die Kanten Feineinstell-Mittel (27) tragen, die sich zwischen oberer Fläche (17A) und unterer Fläche (17B) erstrecken, um die Position der Knochenschraube (45) in bezug auf die Platte festzulegen, und
d) einer Lastübertragungsscheibe (35) zwischen Plattenelement (17) und Knochenschrauben (45), um Last von den Knochenschrauben auf das Plattenelement zu verteilen, wobei die Lastübertragungsscheibe Seitenwandbereiche (39, 40) aufweist, die mit den Feineinstell-Mitteln an den Kanten in Eingriff bringbar sind, und eine Öffnung in der Scheibe (35) vorgesehen ist, um einen Bereich jeder Knochenschraube (45) durch diese hindurchgehend unter einem Winkel in bezug auf die obere Fläche (17A) der Platte zu Positionieren.

29. Knochen-Fixierungsvorrichtung nach einem der Ansprüche 24 - 26, bei welcher das Plattenelement (17) einen asymmetrischen Querschnitt, obere Fläche und untere Fläche und parallele äußere, einander gegenüberliegende Kanten mit einem langgestreckten Schlitz aufweist, welcher von einem Umfangsbereich umgeben ist, welcher die parallelen äußeren, einander gegenüberliegenden Kanten aufweist, und die Lastübertragungsscheibe eine versenkte Öffnung zum Aufnehmen der Lastübertragungsfläche des oberen Endabschnittes der Knochenschraube und zum Positionieren eines Abschnittes der Knochenschraube durch die Öffnung hindurch aufweist und die Öffnung (36) der lastübertragenden Scheibe so ausgeführt ist, daß die Knochenschraube die Scheibe unter einem spitzen Winkel in bezug auf die obere Fläche (17A) der Platte durchsetzt.

## Revendications

1. Appareil de fixation d'os (10) comprenant au moins deux éléments de fixation (11) prévus, à une première extrémité (12), pour être implantés dans le tissu osseux à des positions mutuellement espacées, une plaque (17) ayant deux bords périphériques parallèles opposés et ayant une rainure allongée (20) ménagée dans la dite plaque entre lesdits bords, et un élément de transfert de charge (35) pour répartir la charge de chaque élément de fixation (11) sur la plaque (17), dans lequel l'élément de transfert de charge (35) et la rainure (20) dans la plaque (17) sont prévus chacun pour permettre le positionnement traversant d'une partie de l' élément de fixation (11), et ladite plaque et ledit élément de transfert de charge sont prévus pour coopérer de manière à fournir des moyens de réglage fin de la relation spatiale entre la plaque et l'élément de transfert de charge, caractérisé en ce que l'élément de transfert de charge (35) et lesdits bords périphériques de la plaque (17) sont prévus pour coopérer de manière à fournir les moyens de réglage fin de la relation spatiale entre la plaque et l'élément de transfert de charge.

2. Appareil suivant la revendication 1, dans lequel l'élément de fixation (11) comporte un filetage de vis (13) à ladite première extrémité.

3. Appareil suivant la revendication 1 ou la revendication 2, dans lequel ledit élément de fixation (11) comporte une partie d'extrémité (48) ayant une surface de transfert de charge (51).

4. Appareil suivant la revendication 1 ou la revendication 2, dans lequel ledit élément de fixation comporte une partie filetée (14) pour recevoir par vissage un écrou (31).

5. Appareil suivant une quelconque des revendications 1 à 4, dans lequel ladite plaque (17) présente des surfaces supérieure et inférieure (17A,17B) inclinées l'une par rapport à l'autre.

6. Appareil suivant la revendication 5, dans lequel ledit élément de transfert de charge (35) est prévu pour supporter ledit élément de fixation le long d'une ligne qui forme un angle aigu par rapport à la surface supérieure (17A) de la plaque.

7. Appareil suivant la revendication 1, dans lequel au moins undit élément de fixation (11) comprend une vis d'os ayant une partie centrale (15) qui inclut une surface de transfert de charge, et une partie filetée (14) pour recevoir un écrou (31) par vissage.

8. Appareil de fixation d'os suivant une quelconque des revendications 1 à 7, dans lequel lesdits bords périphériques opposés parallèles portent des moyens de réglage fin s'étendant entre les surfaces supérieure et inférieure pour fixer la position de l'élément de fixation par rapport à la plaque.

9. Appareil suivant une quelconque des revendications 1 à 8, dans lequel ledit élément de transfert de charge (35) comprend des portions de paroi latérale (39,40), pour venir en prise avec les moyens de réglage fin auxdits bords périphériques, et une ouverture (36) pour la mise en place traversante d'une portion dudit élément de fixation (11).

10. Appareil suivant une quelconque des revendications 1 à 9, dans lequel l'élément de transfert de charge comprend une rondelle (35) ayant une première collerette qui s'étend en travers de la rainure entre les bords, et une paire de collerettes en intersection s'étendant suivant des angles par rapport à ces derniers.

11. Appareil suivant une quelconque des revendications 1 à 10, dans lequel la rainure (20) est dimensionnée pour recevoir une paire de moyens de fixation mutuellement espacés et chirurgicalement implantés (11; 45).

12. Appareil suivant une quelconque des revendications 1 à 10, dans lequel les moyens de réglage fin comprennent des dents (27;28) espacées le long des bords.

13. Appareil suivant la revendication 12, dans lequel les moyens de réglage fin comprennent des dents régulièrement espacées (27;28) espacées le long de chacun des bords et des dents correspondantes (37; 38) sur l'élément de transfert de charge (35;52) pour venir en prise avec les dents (27;28 ) des bords.

14. Appareil suivant une quelconque des revendications 1 à 13, dans lequel l'élément de transfert de charge (35;52) comporte une ouverture (36;56;57;58) ayant un centre qui concorde avec l'axe central de la rainure (20;54) pendant l'utilisation.

15. Appareil suivant une quelconque des revendications 1 à 14, dans lequel l'élément de transfert de charge (35;52) comporte un évidement qui s'aligne avec la plaque de sorte que ledit élément (35;52) glisse sur la plaque (17) à l'endroit de l'évidement.

16. Appareil suivant une quelconque des revendications 1 à 15, dans lequel l'élément de transfert de charge comporte trois portions de collerette en intersection incluant une collerette centrale (41) et deux collerettes latérales (60;61) qui sont mutuellement espacées et positionnées de manière à s'appuyer contre les bords de la plaque (17).

17. Appareil suivant une quelconque des revendications 1 à 15, comprenant en outre un écrou (31) à surface inférieure sphérique (32), et l'ouverture de la rondelle de transfert de charge (42) est de configuration sphérique pour recevoir l'écrou.

18. Appareil suivant une quelconque des revendications 1 à 17, dans lequel l'élément de fixation (11) comprend une vis d'os comportant une partie centrale (15) qui présente une surface de transfert de charge profilée s'étendant longitudinalement, et une deuxième partie filetée pour recevoir un écrou (31) par vissage ; et dans lequel ladite plaque présente des surfaces supérieure et inférieure et des bords opposés extérieurs parallèles avec une rainure allongée entourée par une portion périphérique ayant lesdits bords opposés extérieurs parallèles, et la surface inférieure de la plaque est fraisée (26) à l'endroit de la rainure (20) pour s'ajuster à la surface de transfert de charge de la vis d'os, la rainure (20) étant dimensionnée pour recevoir la deuxième partie filetée de la vis d'os.

19. Appareil suivant la revendication 18, dans lequel les bords de la plaque d'os (17) portent des moyens de réglage fin peu espacés (27;28) s'étendant entre les surfaces supérieure et inférieure pour déterminer la position de la vis d'os par rapport à la plaque, et l'espacement desdits moyens autorise plusieurs positions de réglage sur l'épaisseur de la vis d'os.

20. Appareil suivant la revendication 18 ou 19, comprenant en outre un écrou (31) à surface inférieure annulaire courbe (32) et dans lequel l'élément de transfert de charge (35) comporte une ouverture (36) ayant un centre qui concorde avec l'axe central de la rainure pendant l'utilisation, et l'ouverture est configurée en correspondance de l'écrou (31) pour recevoir l'écrou.

21. Appareil suivant la revendication 17 ou 18, comprenant en outre un écrou (31) à surface inférieure sphérique (32) et l'ouverture de l'élément de transfert de charge (56 ; 57 ; 58) est configurée sphériquement pour recevoir l'écrou.

22. Appareil suivant une quelconque des revendications 19 à 21, dans lequel l'ouverture (56;57;58) comporte une partie hémisphérique d'entourage (55) qui reçoit une partie hémisphérique correspondante (32) de l'écrou.

23. Appareil suivant une quelconque des revendication 18 à 22, dans lequel la rainure (20) présente une surface inférieure chanfreinée (26) et la vis d'os présente une surface correspondante qui bute contre la surface chanfreinée pour le transfert de charge.

24. Appareil suivant une quelconque des revendications 1 à 6 ou 8 à 17, dans lequel l'élément de fixation est une vis d'os.

25. Appareil suivant la revendication 24, dans lequel l'élément de transfert de charge (35;52) comprend une surface hémisphérique (55) qui concorde avec la surface de transfert de charge (51) de la vis d'os.

26. Appareil suivant une des revendications 24 ou 25, dans lequel l'élément de transfert de charge (52) comporte une ouverture inclinée (56;57) qui reçoit la vis d'os (45) dans une position inclinée par rapport à la surface supérieure de la plaque.

27. Appareil suivant une quelconque des revendications 24-26, dans lequel la plaque d'os présente des surfaces planes supérieure (17A) et inférieure (17B) qui forment un angle aigu, et des bords opposés extérieurs parallèles, avec une rainure allongée (20) entourée par une partie périphérique ayant lesdits bords opposés extérieurs parallèles, et la rainure de plaque (20) est une rainure inclinée qui est décalée suivant un angle aigu par rapport à la surface supérieure (17A) de la plaque.

28. Appareil de fixation d'os suivant la revendication 27, comprenant :
(a) deux vis d'os (45) ayant chacune une première partie d'extrémité (12) prévue pour être chirurgicalement implantée dans une masse d'os cervical d'un patient à une première et une deuxième positions, mutuellement espacées sur la masse osseuse ;
(b) une plaque (17) présentant des surfaces supérieure et inférieure et des bords opposés extérieurs parallèles avec une rainure longitudinale allongée (20) entourée par une partie périphérique ayant lesdits bords opposés extérieurs parallèles et la plaque étant asymétrique en section transversale ;
(c) les bords portant des moyens de réglage fin (27) qui s'étendent entre les surfaces supérieure (17A) et inférieure (17B) pour fixer la position de la vis d'os (45) par rapport à la plaque (17) ; et
(d) une rondelle de transfert de charge (35) en interface avec la plaque (17) et les vis d'os (45) pour répartir la charge des vis d'os sur la plaque, et comportait des portions de paroi latérale (39,40) de la rondelle, pour venir en prise avec les moyens de réglage fin à l'endroit des bords, et une ouverture dans la rondelle (35) pour positionnement traversant d'une portion de chaque vis d'os (45) suivant un angle par rapport à la surface supérieure (17A) de la plaque.

29. Appareil de fixation d'os suivant une quelconque des revendications 24-26, dans lequel la plaque ( 17) a une section transversale asymétrique, des surfaces supérieure et inférieure, et des bords opposés extérieurs parallèles avec une rainure allongée entourée par une partie périphérique ayant lesdits bords opposés extérieurs parallèles ; la rondelle de transfert de charge ayant une ouverture fraisée pour recevoir la surface de transfert de charge de la partie d'extrémité supérieure de la vis d'os, et pour le positionnement traversant d'une partie de la vis d'os ; ladite ouverture (36) de la rondelle de transfert de charge étant prévue pour permettre à la vis d'os de traverser la rondelle suivant un angle aigu par rapport à la surface supérieure (17A) de la plaque.
